(19) Europäisches Patentamt / European Patent Office / Office européen des brevets

(11) **EP 3 868 285 A1**

(12) **EUROPÄISCHE PATENTANMELDUNG**

(43) Veröffentlichungstag:
**25.08.2021 Patentblatt 2021/34**

(51) Int Cl.:
**A61B 5/0205** (2006.01)  **A61B 5/024** (2006.01)
**A61B 5/08** (2006.01)  **A61B 5/113** (2006.01)

(21) Anmeldenummer: **21156009.9**

(22) Anmeldetag: **09.02.2021**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **14.02.2020 DE 102020103911**

(71) Anmelder: **Technische Hochschule Lübeck**
**23562 Lübeck (DE)**

(72) Erfinder:
• **Kusche, Roman**
 **21075 Hamburg (DE)**
• **Hellbrück, Horst**
 **23562 Lübeck (DE)**

(74) Vertreter: **Wallinger, Michael**
 **Wallinger Ricker Schlotter Tostmann**
 **Patent- und Rechtsanwälte Partnerschaft mbB**
 **Zweibrückenstrasse 5-7**
 **80331 München (DE)**

(54) **VERFAHREN ZUR KONTAKTLOSEN MESSUNG WENIGSTENS EINES VITALPARAMETERS EINES PATIENTEN**

(57) Bei einem Verfahren zur kontaktlosen Messung wenigstens eines Vitalparameters eines Patienten, insbesondere dessen Atemfrequenz und/oder dessen Herzfrequenz, werden gleichzeitig mit zwei verschiedenen kontaktlosen Messverfahren erste und zweite Messwerte an dem Patienten gemessen und daraus der wenigstens eine Vitalparameter bestimmt.

Bei den beiden kontaktlosen Messverfahren handelt es sich insbesondere um ein kapazitives Distanzmessverfahren und um ein Ultraschalldistanzmessverfahren, das jeweils die Bewegungen des Brustkorbs und/oder der Bauchdecke des Patienten misst.

Das Verfahren beinhaltet die Schritte, dass die ersten Messwerte mit den zweiten Messwerten verglichen werden und dass die Güte der zweiten Messwerte unter Verwendung der ersten Messwerte und des Ergebnisses des Vergleichs bewertet wird. Auf diese Weise können ungültige zweite Messwerte als solche erkannt und automatisch verworfen werden. Damit wird die Zuverlässigkeit der kontaktlosen Messung des Vitalparameters des Patienten, insbesondere von dessen Herzfrequenz, erhöht.

Mit einer kapazitiven Distanzmessung kann weiterhin eine einfache und genaue Messung der Atmungsaktivität des Patienten erfolgen.

Fig. 3

**Beschreibung**

Die vorliegende Erfindung betrifft ein Verfahren zur kontaktlosen Messung wenigstens eines Vitalparameters eines Patienten. Der Begriff "Patient" ist dabei nicht einschränkend als kranke oder hilfsbedürftige Person zu verstehen, sondern als Person, an der im Rahmen einer ärztlichen oder sonstigen Dienstleistung, insbesondere einer Heilbehandlung oder einer vorsorglichen Überwachung, eine Messung durchgeführt wird. Der Begriff "Patient" soll außerdem sowohl die männliche als auch die weibliche Form einschließen.

Die Erfindung wird im Folgenden anhand eines menschlichen Patienten beschrieben, kann jedoch auch auf Tiere als Patienten angewandt werden.

Die kontinuierliche Messung von Vitalparametern eines Patienten, insbesondere der Atemfrequenz oder der Herzfrequenz, über einen längeren Zeitraum ist sowohl in der Medizin als auch in anderen Anwendungen und Situationen, wie beim Sport und bei Freizeit- oder Fitnessaktivitäten, im Verkehr, am Arbeitsplatz oder aber im Schlaf von hohem Interesse.

Die zuverlässige Überwachung der Vitalparameter kann für alle Altersgruppen relevant sein. So müssen beispielsweise Frühgeborene in Inkubatoren kontinuierlich überwacht werden. Da mit dem Alter auch das Risiko eines kardiovaskulären Ereignisses, insbesondere eines Notfalls, steigt, sollten andererseits auch die Herz- und Atmungsaktivität von älteren Menschen insbesondere während der Abwesenheit medizinischen Personals kontinuierlich überprüft werden.

Sowohl Herz- als auch Atmungsaktivitäten werden häufig mittels elektrischer Messverfahren bestimmt, welche unter Verwendung von Kontaktelektroden die notwendigen Biosignale vom Körper ableiten, beispielsweise durch ein Elektrokardiogramm (EKG) oder durch Bioimpedanzmessungen.

Für eine kurze Messdauer ist dies häufig akzeptabel. Sollen jedoch, wie zuvor beschrieben, Menschen über längere Zeiträume überwacht werden, ist die Kontaktierung mittels Elektroden ungeeignet. Zudem müssten je nach Messumgebung die Elektroden ggf. von dem - üblicherweise medizinisch ungeschulten - Patienten selbst korrekt auf dessen Körper platziert werden.

Somit ist es von hohem Interesse, Vitalparameter wie Atemfrequenz oder Herzfrequenz kontaktlos an dem Patienten zu messen und physische Zusammenhänge und Plausibilitäten von den Messungen abzuleiten.

Das Wort "kontaktlos" soll im vorliegenden Zusammenhang nicht nur bedeuten, dass ein für das Messverfahren eingesetzter Sensor keinen elektrischen Kontakt mit der Haut des Patienten hat - wie dies beispielsweise für eine Kontaktelektrode erforderlich ist -, sondern dass der Sensor während der Messung von dem Patienten einen Abstand größer 0 haben kann, beispielsweise einen Abstand von 20 cm. Ein Messverfahren, welches einen in oder auf der Kleidung des Patienten angebrachten Sensor einsetzt (sogenannte "Wearables"), ist somit ebenfalls nicht kontaktlos im Sinne der vorliegenden Anmeldung.

Eine in diesem Sinne kontaktlose Messung erhöht den Komfort für den Patienten erheblich, da der Sensor in keiner Weise am Körper des Patienten befestigt oder auf der Körperoberfläche platziert werden muss.

Herkömmliche kontaktlose Messverfahren beruhen oftmals auf Kameratechnik. Diese haben jedoch den Nachteil, dass sie stark in die Privatsphäre des Patienten eingreifen, da der Patient während der Messung kontinuierlich gefilmt wird, und somit ggf. sensible Daten erzeugen. Selbst wenn Letzteres technisch vermieden wird, ist die Akzeptanz durch den Patienten im Allgemeinen nicht hoch. Zudem sind die Kosten und der auftretende Signalverarbeitungsaufwand hoch.

Weitere bekannte kontaktlose Messverfahren beruhen auf der Nutzung elektromagnetischer Felder. Da diese jedoch in die Gruppe 2B der IARC-Skala eingestuft werden, gelten sie als "möglicherweise krebserregend" und sind somit möglichst zu vermeiden.

Aufgrund des Abstands des Sensors vom Körper des Patienten lassen sich durch kontaktlose Messverfahren oftmals nur weniger zuverlässige Messdaten gewinnen als durch kontaktbehaftete Verfahren, insbesondere solche mit Kontaktelektroden als Sensoren.

Weiterhin kann es bei kontaktlosen Messverfahren erforderlich sein, mehrere Sensoren zu verwenden, die auf verschiedenen Seiten des Patienten angeordnet sind. Eine solche Anordnung macht den Messaufbau komplizierter, engt den Patienten ein und beschränkt diesen in seiner Bewegungsfreiheit.

Der vorliegenden Erfindung liegt daher die Aufgabe zugrunde, ein verbessertes Verfahren zur kontaktlosen Messung wenigstens eines Vitalparameters eines Patienten anzugeben.

Diese Aufgabe wird durch ein Messverfahren gemäß Anspruch 1, durch eine Messvorrichtung gemäß Anspruch 11 sowie durch ein weiteres Messverfahren gemäß Anspruch 12 gelöst. Vorteilhafte Weiterbildungen der Erfindung sind in den Unteransprüchen enthalten.

Die Erfindung gemäß den Ansprüchen 1 und 11 beruht auf dem Gedanken, in dem Verfahren zur kontaktlosen Messung wenigstens eines Vitalparameters eines Patienten zwei verschiedene Messverfahren einzusetzen und die Ergebnisse des einen Verfahrens mithilfe der Ergebnisse des anderen Verfahrens zu bewerten und dadurch zu kontrollieren.

Zu diesem Zweck werden in dem erfindungsgemäßen Messverfahren gleichzeitig erste Messwerte mit einem ersten kontaktlosen Messverfahren und zweite Messwerte mit einem zweiten kontaktlosen Messverfahren, welches von dem ersten kontaktlosen Messverfahren verschieden ist, an dem Patienten gemessen, wobei der wenigstens eine Vitalparameter aus den ersten und/oder aus den zweiten Messwerten bestimmt wird. Dann werden die ersten Messwerte mit den zweiten Messwerten verglichen und die Güte der zweiten Messwerte mithilfe der erste Messwerte und des Ergebnisses des Vergleichs bewertet.

Auf diese Weise können die Zuverlässigkeit der zweiten Messwerte, beispielsweise deren Plausibilität, und damit die Zuverlässigkeit der aus den zweiten Messwerten bestimmten Werte des Vitalparameters erhöht werden.

Das erfindungsgemäße Messverfahren wird vorzugsweise bei einem liegenden Patienten angewandt. Dies ist aber nicht zwingend; es ist auch denkbar, dass der Patient bei der Anwendung des Verfahrens sitzt, beispielsweise am Schreibtisch oder am Lenkrad eines Autos, oder eine andere, beispielsweise stehende Position einnimmt.

Vorteile des erfindungsgemäßen Verfahrens zur kontaktlosen Messung wenigstens eines Vitalparameters eines Patienten sind, dass damit Langzeitmessungen möglich sind, dass es eine deutlich höhere Störsicherheit hat als herkömmliche Verfahren, dass es eine günstige Instrumentierung ermöglicht und dass es einfach anwendbar ist, insbesondere dass dafür keine spezielle medizinische Ausbildung notwendig ist.

Das erfindungsgemäße Verfahren kann beispielsweise eingesetzt werden in der Intensivmedizin, in der Neugeborenenmedizin, für Fitness-Anwendungen, für alltagstaugliche Assistenzlösungen für ein selbstbestimmtes Leben ("Ambient Assisted Living", AAL), in Pflegeheimen, zur Arbeitsplatzüberwachung, wobei die Sensoren dann beispielsweise an einem Bildschirm befestigt sind, oder im Auto, wobei die Sensoren dann beispielsweise im Lenkrad integriert sind.

In einer bevorzugten Ausführung der Erfindung ist der wenigstens eine Vitalparameter ein auf die Atmung des Patienten bezogener Parameter, insbesondere die Atemfrequenz, und/oder ein auf die Herzaktivität des Patienten bezogener Parameter, insbesondere die Herzfrequenz.

In dieser Ausführung der Erfindung misst das erste und/oder das zweite kontaktlose Messverfahren bevorzugt Bewegungen des Brustkorbs und/oder der Bauchdecke des Patienten. Da sich der Brustkorb und/oder die Bauchdecke beim Atmen heben und senken und auch durch den Herzschlag in Schwingungen versetzt werden, lassen sich sowohl die Atem- als auch die Herzaktivität anhand der Bewegungen des Brustkorbs und/oder der Bauchdecke detektieren.

Der Brustkorb und/oder die Bauchdecke des Patienten können während der Messungen unbedeckt sein. Da die Bewegungen des Brustkorbs und/oder der Bauchdecke dann unmittelbar anhand der Bewegungen der Körperoberfläche detektiert werden können, ermöglicht dies eine besonders genaue Messung.

Bevorzugt ist jedoch, dass der Brustkorb und/oder die Bauchdecke des Patienten während der Messungen bedeckt sind, insbesondere mit wenigstens einem Kleidungsstück und/oder mit wenigstens einer Decke. Dies erhöht den Komfort des Patienten erheblich, da er sich für die Messungen nicht teilweise entkleiden muss. Außerdem bleibt die Privatsphäre des Patienten auf diese Weise besser gewahrt. Vor allem aber lässt sich das Verfahren damit auch in Situationen anwenden, in denen eine kontinuierliche Verfügbarkeit des Patienten über einen längeren Zeitraum mit unbedecktem Brustkorb und/oder unbedeckter Bauchdecke praktisch nicht machbar ist. Diese umfassen beispielsweise die eingangs erwähnten Situationen, nämlich den Sport, die Freizeit- oder Fitnessaktivitäten, den Verkehr, den Arbeitsplatz oder auch den Schlaf. In allen diesen Situationen ist der Patient bekleidet und/oder, insbesondere mit einer Bettdecke, bedeckt.

Bei der Messung von Bewegungen des Brustkorbs und/oder der Bauchdecke des Patienten ist es besonders bevorzugt, dass die ersten Messwerte und/oder die zweiten Messwerte Abstandswerte für einen Abstand zwischen einem Sensor und der Oberfläche des Körpers des Patienten, ggf. inklusive dessen Kleidung oder Bedeckung, sind. Da die Bewegungen des Brustkorbs und/oder der Bauchdecke, insbesondere dessen Heben und Senken beim Atmen bzw. dessen durch den Herzschlag verursachte Schwingungen, eine veränderte räumliche Lage der Oberfläche des Körpers des Patienten bzw. dessen Kleidung oder Bedeckung zur Folge haben, besteht hierdurch ein direkter Zusammenhang zwischen dem genannten Abstand und der zu messenden Atem- bzw. Herzaktivität des Patienten.

Die Amplitude typischer Bewegungen des Brustkorbs und/oder der Bauchdecke beim Atmen liegt im Bereich von 4 bis 12 mm, die Amplitude typischer Bewegungen des Brustkorbs und/oder der Bauchdecke infolge der Herzschläge dagegen nur im Bereich von 0,2 bis 0,5 mm.

Das erfindungsgemäße Verfahren erlaubt in einer bevorzugten Ausführung, dass ein Sensor für das erste kontaktlose Messverfahren und/oder ein Sensor für das zweite kontaktlose Messverfahren während der Messung einen Abstand von wenigstens 10 cm, vorzugsweise wenigstens 15 cm, weiter vorzugsweise wenigstens 20 cm von der Oberfläche des Körpers des Patienten bzw. dessen Kleidung oder Bedeckung aufweist. Damit ist in vielen Fällen eine ausreichende Bewegungsfreiheit des Patienten in der jeweiligen Anwendungssituation, beispielsweise am Steuer eines Fahrzeugs, am Schreibtisch oder auch im Bett, gegeben.

In einer besonders bevorzugten Ausführung der Erfindung ist das erste kontaktlose Messverfahren ein kapazitives Messverfahren und das zweite kontaktlose Messverfahren ein Ultraschallmessverfahren.

Wie die Erfinder erkannt haben, kann mittels einer kapazitiven Distanzmessung die Atmung detektiert werden.

Hierbei wird, vom Körper des Patienten beanstandet, ein zwei Elektroden aufweisender Sensor angeordnet, zwischen denen ein elektrisches Feld mit gekrümmten, in den Raum hineinreichenden Feldlinien erzeugt wird. Die beiden Elektroden bilden somit gemeinsam mit der umgebenden Luft als Dielektrikum einen Kondensator. Da die Permittivität des menschlichen Körpers eine andere ist als die der Luft, ändert sich die Kapazität dieses Kondensator, sobald der Patient in die Nähe der Elektroden gelangt, wobei die Kapazität vom Abstand der Körperoberfläche des Patienten von den Elektroden abhängt. Auf diese Weise können der Abstand der Körperoberfläche des Patienten von dem kapazitiven Sensor und damit die Bewegungen des Brustkorbs und/oder der Bauchdecke ermittelt und somit die Atmung des Patienten detektiert werden.

Von besonderem Vorteil ist bei diesem Verfahren, dass das elektrische Verhalten, insbesondere die Permittivität, von Textilien dem der Luft deutlich mehr ähnelt als dem des menschlichen Körpers. Somit kann die Atmung durch den kapazitiven Sensor auch durch Textilien hindurch zuverlässig detektiert werden.

Besonders bevorzugt ist das zweite kontaktlose Messverfahren ein Ultraschallmessverfahren, insbesondere ein Messverfahren zur Distanzmessung mittels Ultraschall. Dazu wird ein Schallsignal von einem Aktuator ausgegeben und von einem Sensor aufgenommen. Es kann sich bei Aktuator und Sensor auch um dasselbe Bauteil handeln. Die interessante Messgröße ist die Laufzeit des Ultraschallsignals.

Unter der Annahme, dass ein Ultraschallsignal in die Richtung des Patienten gesendet, an dessen Körperoberfläche reflektiert und von einem Sensor detektiert wird, lässt sich aus der Signallaufzeit und der Kenntnis der Schallgeschwindigkeit in der Luft die zurückgelegte Strecke bestimmen. Da die Geometrie des Brustkorbs und/oder der Bauchdecke des Patienten sowohl durch das Atmen als auch durch den Herzschlag zeitlich variiert wird, ändert sich dadurch auch die Distanz zwischen dem Aktuator bzw. Sensor und der reflektierenden Körperoberfläche. Auf diese Weise kann sowohl die Atem- als auch die Herzaktivität des Patienten durch das Ultraschallmessverfahren gemessen werden.

In der Praxis herrschen allerdings oftmals nicht die für das Ultraschallmessverfahren idealen Messbedingungen vor. So ist häufig der Oberkörper des Patienten durch Textilien wie Kleidung oder eine Bettdecke verdeckt. Somit wird ein Großteil des Ultraschallsignals nicht an der eigentlichen Körperoberfläche reflektiert, sondern an der Oberfläche des Textils. Da dieses in der Regel auf dem Brustkorb bzw. auf der Bauchdecke aufliegt, können unter günstigen Umständen weiterhin Atmung und Herzschlag mittels Ultraschall detektiert werden. Unter ungünstigen Umständen liefern die empfangenen Schallsignale jedoch Fehlinformationen.

Hier setzt die vorliegende Erfindung an, indem sie das erste kontaktlose Messverfahren, vorzugsweise ein kapazitives Messverfahren, zur Bestimmung und Verringerung der Unsicherheit über die genannte Zuverlässigkeit des zweiten kontaktlosen Messverfahrens, vorzugsweise eines Ultraschallmessverfahrens, für einen Vergleich der ersten mit dem zweiten Messwerten heranzieht.

In einer besonders bevorzugten Ausführung der Erfindung weist der Vergleich der ersten Messwerte mit den zweiten Messwerten den Schritt einer Ermittlung eines Grades einer Übereinstimmung zwischen einem ersten Frequenzanteil der ersten Messwerte und einem zweiten Frequenzanteil der zweiten Messwerte auf.

Hierdurch kann der Tatsache Rechnung getragen werden, dass verschiedene Vitalparameter aus verschiedenen Frequenzanteilen der ersten und/oder der zweiten Messwerte abgeleitet werden können.

Insbesondere können die ersten und die zweiten Messwerte insgesamt unterschiedlich sein, beispielsweise aufgrund verschiedener Auflösungen der beiden kontaktlosen Messverfahren, während bestimmte Frequenzanteile der ersten bzw. der zweiten Messwerte jedoch übereinstimmen, da sie denselben Vitalparameter repräsentieren.

Um die Zuverlässigkeit der zweiten Messwerte mithilfe der ersten Messwerte zu überprüfen, kann es daher sinnvoll sein, nur eine bestimmte Frequenzkomponente der ersten und der zweiten Messwerte - insbesondere eine denselben Vitalparameter repräsentierende Frequenzkomponente - auf Übereinstimmung zu prüfen.

In einer besonders bevorzugten Variante dieser Ausführung ist der erste Frequenzanteil der ersten Messwerte identisch mit den ersten Messwerten selbst, und der zweite Frequenzanteil der zweiten Messwerte ist ein niederfrequenter Anteil, insbesondere mit einer Frequenz $f < 1$ Hz, der zweiten Messwerte. Der Begriff "Frequenzanteil" ist somit so zu verstehen, dass nicht unbedingt nur ein Teilbereich der Frequenzen der jeweiligen Messwerte betrachtet wird, sondern dass der Frequenzanteil auch mit den gesamten Messwerten identisch sein kann.

Insbesondere kann es sich bei den ersten Messwerten um die Messwerte des kapazitiven Messverfahrens handeln, welches aufgrund seiner relativ geringen Auflösung nur die Bewegungen des Brustkorbs und/oder der Bauchdecke mit größerer Amplitude und damit nur die Atembewegungen mit ihrer niedrigen Frequenz erfassen kann.

Insbesondere kann es sich bei den zweiten Messwerten um die Messwerte des Ultraschallmessverfahrens handeln, welches aufgrund seiner relativ hohen Auflösung sowohl die Atembewegungen des Brustkorbs und/oder der Bauchdecke mit größerer Amplitude als auch die Schwingungen des Brustkorbs und/oder der Bauchdecke mit kleinerer Amplitude infolge der Herzschläge erfassen kann.

Um aus den zweiten Messwerten das Signal für die Atembewegungen des Brustkorbs und/oder der Bauchdecke zu isolieren, wird der zweite Frequenzanteil der zweiten Messwerte durch einen Tiefpassfilter bestimmt. Die Frequenz $f$ des zweiten Frequenzanteils ist vorzugsweise so gewählt, dass sie im Bereich einer typischen Atemfrequenz liegt, beispielsweise zwischen 0,2 und 0,5 Hz.

In einer besonders bevorzugten Variante dieser Ausführung der Erfindung wird aus den ersten Messwerten ein auf die Atmung des Patienten bezogener Parameter, insbesondere dessen Atemfrequenz, und aus einem höherfrequenten Anteil, insbesondere mit einer Frequenz f > 1 Hz, der zweiten Messwerte ein auf die Herzaktivität des Patienten bezogener Parameter, insbesondere dessen Herzfrequenz, ermittelt.

Wenn das erste kontaktlose Messverfahren, insbesondere das Kapazitätsmessverfahren, die Atmung zuverlässig detektiert, können die ersten Messwerte genutzt werden, um Aussagen über die Qualität der zweiten Messwerte aus dem gleichzeitig ausgeführten zweiten Messverfahren, insbesondere der Ultraschallmessung, zu treffen.

Besonders bevorzugt werden dazu die ersten Messwerte (insbesondere aus der kapazitiven Messung) mit dem niederfrequenten Signalanteil der zweiten Messwerte (insbesondere aus der Ultraschallmessung) verglichen. Bei hoher Übereinstimmung bzw. guter Korrelation können die zweiten Messwerte als zuverlässig angesehen werden. Dann können auch die höherfrequenten Signalanteile (insbesondere mit f > 1 Hz) der zweiten Messwerte als zuverlässige Messwerte für den Herzschlag angesehen werden. Bei niedriger Übereinstimmung bzw. schlechter Korrelation können die zweiten Messwerte dagegen verworfen werden.

Die in dem erfindungsgemäßen Messverfahren eingesetzten Sensoren können auch andere als kapazitive Sensoren oder Ultraschallsensoren sein, beispielsweise Sensoren, die auf optischen, elektromagnetischen und/oder magnetischen Effekten oder Verfahren basieren.

Es können statt eines einzigen Sensors auch mehrere gleichartige Sensoren verwendet werden, um eine räumliche Auflösung zu erhalten.

Eine erfindungsgemäße Messvorrichtung zur Ausführung des erfindungsgemäßen Messverfahrens weist wenigstens einen ersten Sensor zur Aufnahme der ersten Messwerte, wenigstens einen zweiten Sensor zur Aufnahme der zweiten Messwerte, eine Vergleichseinrichtung zum Vergleichen der ersten Messwerte mit den zweiten Messwerten und eine Bewertungseinrichtung zur Bewertung der Güte der zweiten Messwerte unter Verwendung der ersten Messwerte und des Ergebnisses des Vergleichs auf.

Für die Funktionsweise der einzelnen Komponenten der erfindungsgemäßen Messvorrichtung wird auf die obige ausführliche Beschreibung der entsprechenden Schritte des erfindungsgemäßen Messverfahrens verwiesen.

Ein weiteres erfindungsgemäßes Messverfahren dient zur kontaktlosen Messung wenigstens eines auf die Atmung bezogenen Parameters eines Patienten, insbesondere dessen Atemfrequenz, und ist ein kapazitives Abstandsmessverfahren. Für das Messverfahren wird ein kapazitiver Sensor auf einer einzigen Seite des Patienten in einem Abstand von vorzugsweise wenigstens 10 cm, weiter vorzugsweise wenigstens 15 cm, noch weiter vorzugsweise wenigstens 20 cm von der Oberfläche des Körpers des Patienten, ggf. inklusive dessen Kleidung oder Bedeckung, angeordnet. Dann wird der Abstand zwischen dem kapazitiven Sensor und dem Brustkorb und/oder der Bauchdecke des Patienten, ggf. inklusive der Kleidung oder Bedeckung des Patienten im Bereich des Brustkorbs und/oder der Bauchdecke, im zeitlichen Verlauf gemessen. Schließlich wird aus den Messwerten der auf die Atmung bezogene Parameter bestimmt.

Auch hierzu wird auf die obige, ausführliche Beschreibung des kapazitiven Abstandsmessverfahrens verwiesen.

Weitere Vorteile, Merkmale und Anwendungsmöglichkeiten der vorliegenden Erfindung ergeben sich aus der nachfolgenden, beispielhaften Beschreibung in Zusammenhang mit den Figuren. Es zeigen:

Fig. 1    ein Prinzipschaubild der kapazitiven Distanzmessung;

Fig. 2    ein Prinzipschaubild der Ultraschalldistanzmessung;

Fig. 3    einen beispielhaften Messaufbau für das erfindungsgemäße Messverfahren mit simultaner Abstandsbestimmung mittels kapazitiver Messung und Ultraschallmessung;

Fig. 4    ein Blockschaubild der Signalverarbeitung bei dem erfindungsgemäßen Messverfahren.

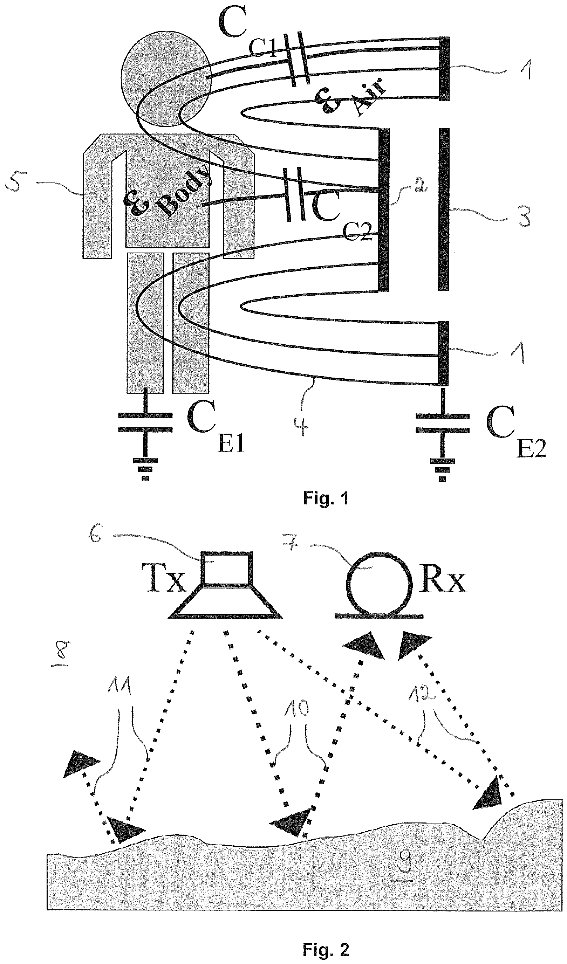

Fig. 1 zeigt ein Prinzipschaubild der kapazitiven Distanzmessung. Auf der rechten Seite ist der Querschnitt eines ringförmigen Sensors dargestellt, welcher aus einem negativen, äußeren Elektrodenring 1 und einer positiven, inneren, kreisscheibenförmigen Elektrode 2 besteht. Zusätzlich ist eine optional einsetzbare aktive Abschirmung 3 dargestellt, welche verwendet werden kann, um die Ausbreitung des elektrischen Feldes nur in eine Richtung zu erzwingen.

Die halbellipsenförmigen Linien stellen die stark vereinfachten elektrischen Feldlinien 4 dar, welche die Luft mit einer relativen Permittivität von $\varepsilon_{Air} \approx 1$ und den Körper des Patienten 5 mit $\varepsilon_{Body} \approx 10^2 \ldots 10^5$ durchdringen. Da die Kapazität $C_{Sensor}$ zwischen der positiven Elektrode 2 und der negativen Elektrode 1 des Sensors 1, 2, 3 von der Permittivität zwischen den Elektroden 1, 2 abhängt ($C_{Sensor} = f(\varepsilon)$), folgt unmittelbar, dass deutlich verschiedene Permittivitäten die gemessene Kapazität $C_{Sensor}$ beeinflussen.

Der zweite physikalische Effekt ist das Vorhandensein von parasitären Kapazitäten. Die Kombination aus leitenden Sensorelektroden 1, 2, leitendem menschlichem Gewebe 5 und der isolierenden Luft dazwischen verursacht die Koppelkapazitäten $C_{C1}$ und $C_{C2}$, welche von dem Abstand zwischen dem Patienten 5 und dem Sensor 1, 2, 3 und weiteren Geometrien abhängen. Daher generiert auch dieser Effekt Informationen. Zusätzliche Koppelkapazitäten $C_E$ zwischen dem Patienten 5 und der Erde ($C_{E1}$), aber auch zwischen dem Sensorsystem 1, 2, 3 und der Erde ($C_{E2}$), sind unvermeidlich. Da diese Kapazitäten von komplexen Geometrien und vielen anderen Bedingungen abhängen, ändern sich die zugehörigen Werte über die Zeit, und es ist unmöglich, diese zu berechnen oder vorherzusagen. Ein typischer Wert aus der Literatur für eine grobe Schätzung ist $C_E \approx 100$ pF. Nimmt man realistische Elektrodengeometrien im cm²-Bereich mit Abständen dazwischen von mehreren cm an, ist die ideale Kapazität von $C_{Sensor}$ in einem sehr kleinen pF-Bereich und daher signifikant kleiner als diese parasitären Effekte. Da die Verbindungskabel und die Messschaltungsbauteile zusätzliche, unbekannte Kapazitäten mit viel höheren Werten erzeugen, sind absolute Messungen ohne Nutzen. Stattdessen werden die parasitären Effekte als konstant angenommen und die schwankenden Veränderungen von $C_{Sensor}$ ermittelt.

Um die resultierende Kapazität $C_{Sensor}$ einschließlich der beschriebenen Effekte zu messen, gibt es mehrere Möglichkeiten. Eine vielversprechende Methode besteht in der Bestimmung der zugehörigen elektrischen Reaktanz $X(C_{Sensor})$, wie in der folgenden Gleichung definiert:

$$X(C_{Sensor}) = 1 / (2\pi f\, C_{Sensor}).$$

Technisch kann $X(C_{Sensor})$ bestimmt werden, indem man eine Wechselspannung anlegt und den auftretenden elektrischen Strom misst. Um danach die Nutzinformation von Signalstörungen zu trennen, ist die Verwendung einer bekannten Erregungsfrequenz vorteilhaft. Die Wahl von hohen Erregungsfrequenzen mit f >> 10 kHz und Signalamplituden im V-Bereich führen zu Strömen im µA-Bereich, welche bequem messbar sind.

Fig. 2 zeigt ein Prinzipschaubild der Ultraschalldistanzmessung. Die Ultraschalldistanzmessung basiert üblicherweise darauf, die Laufzeit eines zwischen einem Ultraschallsender 6 (Tx) und einem Ultraschallempfänger 7 (Rx) übertragenen Pulses zu bestimmen. Bei bekannter Schallgeschwindigkeit $c_{US,Luft}$ in der Luft 8 wird die gemessene Ausbreitungszeit verwendet, um den Abstand zwischen der Tx/Rx-Kombination und dem reflektierenden Gewebe 9 oder einem anderen reflektierenden Material, insbesondere der Kleidung oder einer Bedeckung des Patienten, zu berechnen.

Da die reflektierende Oberfläche normalerweise nicht perfekt eben ist, entstehen für die Reflexion der Ultraschallwellen zusätzliche Wege 11, 12 (in Fig. 2 durch die Pfeile links und rechts dargestellt) neben dem direkten Weg 10 (in Fig. 2 durch die Pfeile in der Mitte dargestellt). Dies führt zu einer Überlagerung von mehreren Signalkomponenten beim Empfänger 7. Um den direkten Weg 10 von den Signalen der anderen Wege 11, 12 zu unterscheiden, übertragen viele Implementierungen kurze Schallpulse und betrachten die zuerst empfangene Komponente als den direkten Weg 10.

Bei Anwendungen mit mehreren aktiven Sendern 6 ist eine klare Zuordnung des Ursprungs des empfangenen Signals schwierig. Zusätzlich begrenzt die Erzeugung von sehr kurzen schmalbandigen Übertragungspulsen die maximale lokale Auflösung, die erreicht werden kann. Ein Ansatz, um dies zu umgehen, ist der Einsatz einer Frequenztrennung über einen kontinuierlichen aktiven Sender.

Berücksichtigt man, dass das empfangene Signal die Überlagerung aller reflektierten Signalkomponenten beim Empfänger 7 ist, so ist die Verarbeitung eines kontinuierlichen, sinusförmigen Signals Rx(t) weniger komplex, und eine Mittelwertbildung verbessert die Signalqualität. Die Amplitude Â sowie die Phasenverschiebung φ von Rx(t) enthält Informationen über den Abstand d zwischen dem Sensor und der reflektierenden Oberfläche, wie in der folgenden Gleichung ausgedrückt, wobei f die Frequenz der Ultraschallsignale ist:

$$Rx(t) = \hat{A} \cdot \sin(2\pi ft + \varphi).$$

Wir nehmen an, dass der absolute Abstand d zwischen dem Gewebe 9 des Patienten und dem Sensor 6, 7 viel größer ist als die winzigen Vibrationen von dessen Brust im Sub-Millimeter-Bereich. Daher sind die Veränderungen von Â, welche durch die Atmung oder durch den Herzschlag verursacht werden, sehr klein. Zusätzlich kann Â sich infolge der Überlagerung der reflektierten Signalkomponenten zufällig verändern. Betrachtet man eine typische Ultraschallfrequenz von $f_{US} = 40$ kHz, so sind die entsprechenden Wellenlängen Aus sehr nah an den Abmessungen der Vibrationen, wie in der folgenden Gleichung abgeschätzt:

$$\lambda_{US} = c_{US,Luft} / f_{US} \approx 343\ \text{m/s} / 40\ \text{kHz} \approx 8{,}6\ \text{mm}.$$

Daher ist die Phasenverschiebung φ des empfangenen Signals sehr empfindlich hinsichtlich geringer Abstandsänderungen Δd, unabhängig vom Gesamtabstand d. Dabei kann vernachlässigt werden, dass der Abstand d selbst unbekannt ist, da er in der vorliegenden Anwendung nicht von Interesse ist.

Es wird daher vorzugsweise die Phasenverschiebung verwendet, um Informationen über die Vibrationen des Brustkorbs und/oder der Bauchdecke zu erhalten, welche durch die Atmung und durch Herzaktivitäten verursacht werden. Insbesondere wird dazu die relative Phasenverschiebung aus dem gesendeten und dem empfangenen Signals berechnet.

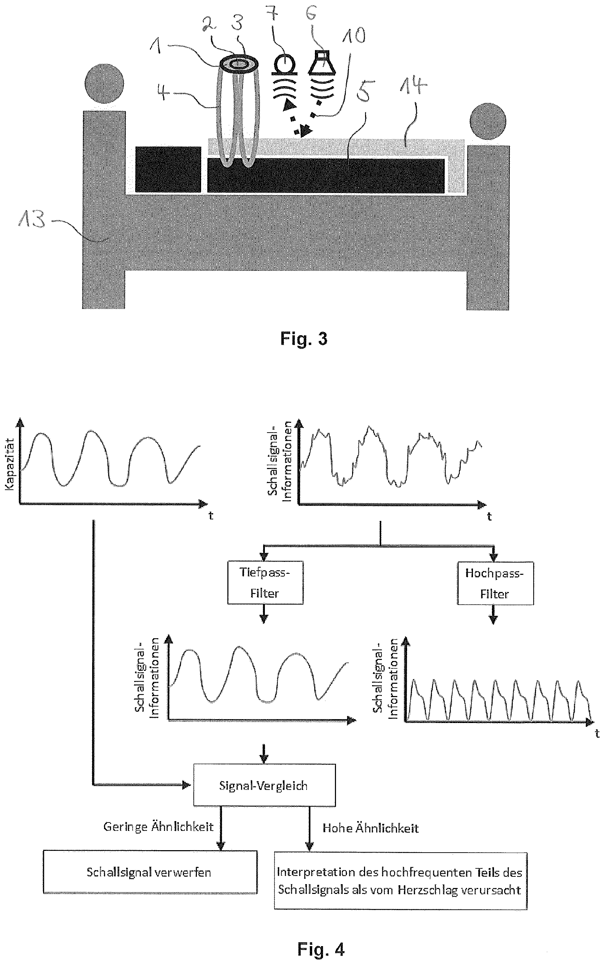

Fig. 3 zeigt einen beispielhaften Messaufbau für das erfindungsgemäße Messverfahren mit simultaner Abstandsbestimmung mittels kapazitiver Messung und Ultraschallmessung.

Hierbei liegt der Patient 5 auf dem Rücken im Bett 13 und ist mit einer Bettdecke 14 bedeckt. Es kann sich bei dem Patienten 5 beispielsweise um einen Patienten in einem Krankenhausbett oder um einen Säugling in einem Inkubator handeln.

Oberhalb des Bettes 13, vorzugsweise von der Decke des Raumes abgehängt, sind in geringem Abstand, insbesondere etwa 20 cm, über der Bettdecke 14 und auf Höhe des Brustkorbs und/oder der Bauchdecke des Patienten 5 ein kapazitiver Sensor 1, 2, 3 (vgl. Fig. 1) für die kapazitive Messung sowie ein aus Sender 6 und Empfänger 7 bestehender Ultraschallsensor für die Ultraschallmessung angebracht. Angedeutet sind in Fig. 3 weiterhin Feldlinien 4 des von dem kapazitiven Sensor erzeugten elektrischen Feldes sowie die von dem Ultraschallsender 6 erzeugten Ultraschallwellen und der direkte Weg 10 dieser Wellen zu dem Ultraschallempfänger 7. Bei dieser Anordnung hat der Patient 5 noch eine ausreichende Bewegungsfreiheit und wird in seinen Schlafgewohnheiten nur unwesentlich beeinträchtigt.

Fig. 4 zeigt ein Blockschaubild der Signalverarbeitung bei dem erfindungsgemäßen Messverfahren.

In dem erfindungsgemäßen Verfahren wird mit dem in Fig. 3 gezeigten Messaufbau der zeitliche Verlauf der Bewegungen des Brustkorbs und/oder der Bauchdecke des durch eine Bettdecke 14 bedeckten Patienten 5 sowohl durch den kapazitiven Sensor 1, 2, 3 als auch durch den Ultraschallsensor 6, 7 gemessen. Die entsprechenden Signalverläufe sind in Fig. 4 oben links für den kapazitiven Sensor 1, 2, 3 und oben rechts für den Ultraschallsensor 6, 7 dargestellt.

Um Einflüsse aus der Verzögerung der Filter zu vermeiden, wurden Nullphasenfilter gewählt.

Das empfangene Signal des Ultraschallsensors 6, 7 ist ein hochfrequenter Sinus (Trägersignal). Von Interesse sind jedoch hauptsächlich die zeitlichen Änderungen der Amplitude und der Phase des Signals. Diese Änderungen haben wiederum Frequenzanteile zwischen ca. 0,1 Hz und 10 Hz (vgl. die "Schallsignalinformationen" in Fig. 4 oben rechts). Um zwischen der langsamen Atmung und dem schnellen Herzschlag unterscheiden zu können, wird nun dieser Frequenzbereich mittels eines Tiefpassfilters und eines Hochpassfilters in zwei Teile unterteilt.

Im Ausführungsbeispiel wird das Signal des Ultraschallsensors 6, 7 digital hochpassgefiltert ($N = 2$, $f_c = 1,2$ Hz) und tiefpassgefiltert ($N = 2$, $f_c = 6$ Hz). Die resultierenden Frequenzanteile sind in Fig. 4 in der Mitte dargestellt. Das tiefpassgefilterte Ultraschallsignal stellt dann eine gute Repräsentation der Atemaktivität des Patienten 5 dar, und das hochpassgefilterte Ultraschallsignal stellt eine gute Repräsentation der Herzaktivität des Patienten 5 dar.

Das Signal des kapazitiven Sensors 1, 2, 3 stellt auch ohne Filterung nach bestimmten Frequenzanteilen eine gute Repräsentation der Atemaktivität des Patienten 5 dar.

Dennoch sind derartige Messergebnisse des Ultraschallsensors 6, 7 nicht in jeder Messsituation zu erzielen, was unter anderen durch die komplexen Überlagerungen der reflektierten kontinuierlichen Schallwellen verursacht wird.

Andererseits reicht die Auflösung des kapazitiven Sensors nicht aus, um die geringen Schwingungen des Brustkorbs und/oder der Bauchdecke infolge des Herzschlags zu detektieren.

Das erfindungsgemäße Verfahren verwendet nun die Signale des kapazitiven Sensors 1, 2, 3, um die Güte des Ultraschallsignals zu bewerten. Dies geschieht durch einen Signalvergleich (in Fig. 4 unten dargestellt) zwischen dem Signal des kapazitiven Sensors 1, 2, 3 und dem tiefpassgefilterten Signal des Ultraschallsensors 6, 7 mithilfe von üblichen, dem Fachmann bekannten Signalvergleichsmethoden. Der Signalvergleich liefert eine Bewertungszahl, welche ein Maß für die Ähnlichkeit der beiden verglichenen Signale darstellt.

Ergibt der Signalvergleich eine hohe Ähnlichkeit der beiden Signale (Bewertungszahl oberhalb eines bestimmten Schwellwertes), so wird das Ultraschallsignal als gültig akzeptiert, somit auch der hochfrequente Anteil des Ultraschallsignals als Repräsentation des Herzschlags des Patienten 5 akzeptiert und damit der hochfrequente Anteil des Ultraschallsignals als durch den Herzschlag des Patienten 5 verursacht interpretiert (Fig. 4 rechts unten).

Ergibt der Signalvergleich dagegen eine nur geringe Ähnlichkeit der beiden Signale (Bewertungszahl unterhalb des Schwellwertes), so wird das Ultraschallsignal nicht als Repräsentation des Herzschlags des Patienten 5 akzeptiert und daher das Ultraschallsignal verworfen (Fig. 4 unten links).

Auf diese Weise wird in das Messverfahren eine zusätzliche Kontrollmöglichkeit integriert, um ungültige Ultraschallmesswerte als solche zu erkennen und vor einer Auswertung, welche zu ungültigen Darstellungen der Herzaktivität des Patienten 5 führen würde, automatisch zu verwerfen. Damit wird die Zuverlässigkeit der kontaktlosen Messung des Herzschlags des Patienten weiter erhöht.

Gleichzeitig erfolgt mit der kapazitiven Distanzmessung eine einfache und genaue Messung der Atmungsaktivität des Patienten 5.

**Bezugszeichenliste**

1 Äußerer Elektrodenring
2 Innere Elektrode
3 Aktive Abschirmung
4 Elektrische Feldlinie
5 Patient
6 Ultraschallsender
7 Ultraschallempfänger
8 Luft
9 Gewebe
10 Direkter Weg der Ultraschallreflexion
11, 12 Zusätzlicher Weg der Ultraschallreflexion
13 Bett
14 Bettdecke

**Patentansprüche**

1. Verfahren zur kontaktlosen Messung wenigstens eines Vitalparameters eines Patienten (5), **dadurch gekennzeichnet, dass** gleichzeitig erste Messwerte mit einem ersten kontaktlosen Messverfahren und zweite Messwerte mit einem zweiten kontaktlosen Messverfahren, welches von dem ersten kontaktlosen Messverfahren verschieden ist, an dem Patienten (5) gemessen werden und dass der wenigstens eine Vitalparameter aus den ersten und/oder aus den zweiten Messwerten bestimmt wird, wobei das Verfahren den Schritt eines Vergleichs der ersten Messwerte mit den zweiten Messwerten und den Schritt einer Bewertung der Güte der zweiten Messwerte unter Verwendung der ersten Messwerte und des Ergebnisses des Vergleichs aufweist.

2. Verfahren gemäß Anspruch 1, **dadurch gekennzeichnet, dass** der wenigstens eine Vitalparameter ein auf die Atmung des Patienten (5) bezogener Parameter und/oder ein auf die Herzaktivität des Patienten (5) bezogener Parameter ist.

3. Verfahren gemäß Anspruch 2, **dadurch gekennzeichnet, dass** das erste und/oder das zweite kontaktlose Messverfahren Bewegungen des Brustkorbs und/oder der Bauchdecke des Patienten (5) misst.

4. Verfahren gemäß Anspruch 3, **dadurch gekennzeichnet, dass** der Brustkorb und/oder die Bauchdecke des Patienten (5) während der Messungen unbedeckt oder, insbesondere mit wenigstens einem Kleidungsstück und/oder mit wenigstens einer Decke (14), bedeckt sind.

5. Verfahren gemäß einem der Ansprüche 3 oder 4, **dadurch gekennzeichnet, dass** die ersten Messwerte und/oder die zweiten Messwerte Abstandswerte für einen Abstand und der Oberfläche des Körpers des Patienten (5), ggf. inklusive dessen Kleidung oder Bedeckung (14), sind.

6. Verfahren gemäß einem der vorhergehenden Ansprüche, **dadurch gekennzeichnet, dass** ein Sensor (1, 2, 3) für das erste kontaktlose Messverfahren und/oder ein Sensor (6, 7) für das zweite kontaktlose Messverfahren während der Messung einen Abstand von wenigstens 10 cm, vorzugsweise wenigstens 15 cm, weiter vorzugsweise wenigstens 20 cm von der Oberfläche des Körpers des Patienten (5), ggf. inklusive dessen Kleidung oder Bedeckung (14), aufweist.

7. Verfahren gemäß einem der vorhergehenden Ansprüche, **dadurch gekennzeichnet, dass** das erste kontaktlose Messverfahren ein kapazitives Messverfahren und das zweite kontaktlose Messverfahren ein Ultraschallmessverfahren ist.

8. Verfahren gemäß Anspruch 1, **dadurch gekennzeichnet, dass** der Vergleich der ersten Messwerte mit den zweiten Messwerten den Schritt einer Ermittlung eines Grades einer Übereinstimmung zwischen einem ersten Frequenzanteil der ersten Messwerte und einem zweiten Frequenzanteil der zweiten Messwerte aufweist.

9. Verfahren gemäß Anspruch 8, **dadurch gekennzeichnet, dass** der erste Frequenzanteil der ersten Messwerte identisch mit den ersten Messwerten selbst ist und der zweite Frequenzanteil der zweiten Messwerte ein niederfrequenter Anteil, insbesondere mit einer Frequenz f < 1 Hz, der zweiten Messwerte ist.

10. Verfahren gemäß Anspruch 9, **dadurch gekennzeichnet, dass** aus den ersten Messwerten ein auf die Atmung des Patienten (5) bezogener Parameter, insbesondere dessen Atemfrequenz, und aus einem höherfrequenten Anteil, insbesondere mit einer Frequenz f > 1 Hz, der zweiten Messwerte ein auf die Herzaktivität des Patienten (5) bezogener Parameter, insbesondere dessen Herzfrequenz, ermittelt wird.

11. Messvorrichtung zur Ausführung eines Messverfahrens gemäß einem der vorhergehenden Ansprüche, aufweisend wenigstens einen ersten Sensor (1, 2, 3) zur Aufnahme der ersten Messwerte, wenigstens einen zweiten Sensor (6, 7) zur Aufnahme der zweiten Messwerte, eine Vergleichseinrichtung zum Vergleichen der ersten Messwerte mit den zweiten Messwerten und eine Bewertungseinrichtung zur Bewertung der Güte der zweiten Messwerte unter Verwendung der ersten Messwerte und des Ergebnisses des Vergleichs.

12. Verfahren zur kontaktlosen Messung wenigstens eines auf die Atmung bezogenen Parameters eines Patienten (5), insbesondere dessen Atemfrequenz, **dadurch gekennzeichnet, dass** das kontaktlose Messverfahren ein kapazitives Abstandsmessverfahren ist, wobei ein kapazitiver Sensor (1, 2, 3) auf einer einzigen Seite des Patienten (5) in einem Abstand von vorzugsweise wenigstens 10 cm, weiter vorzugsweise wenigstens 15 cm, noch weiter vorzugsweise wenigstens 20 cm von der Oberfläche des Körpers des Patienten (5), ggf. inklusive dessen Kleidung oder Bedeckung (14), angeordnet wird, der Abstand zwischen dem kapazitiven Sensor (1, 2, 3) und dem Brustkorb und/oder der Bauchdecke des Patienten (5), ggf. inklusive der Kleidung oder Bedeckung (14) des Patienten (5) im Bereich des Brustkorbs und/oder der Bauchdecke, im zeitlichen Verlauf gemessen wird und aus den Messwerten der auf die Atmung bezogene Parameter bestimmt wird.

**Fig. 1**

**Fig. 2**

Fig. 3

Fig. 4

Europäisches
Patentamt

European
Patent Office

Office européen
des brevets

## EUROPÄISCHER RECHERCHENBERICHT

**Nummer der Anmeldung**

EP 21 15 6009

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y | DE 10 2011 112226 A1 (LIFETAIX GMBH [DE]) 8. Mai 2013 (2013-05-08)<br>* Absatz [0003] *<br>* Absatz [0009] - Absatz [0014] *<br>* Absatz [0032] - Absatz [0038] *<br>* Abbildungen 1-7 *<br>* Anspruch 19 *<br>----- | 1-5,8-11<br><br>6,7 | INV.<br>A61B5/0205<br>A61B5/024<br>A61B5/08<br>A61B5/113 |
| X<br><br><br><br><br><br><br><br>Y | ANDREAS BRAUN ET AL: "Capacitive proximity sensing in smart environments", JOURNAL OF AMBIENT INTELLIGENCE AND SMART ENVIRONMENTS,<br>Bd. 7, Nr. 4, 13. Juli 2015 (2015-07-13), Seiten 483-510, XP055466832,<br>The Netherlands<br>ISSN: 1876-1364, DOI: 10.3233/AIS-150324<br>* Seite 4, linke Spalte, letzter Absatz - rechte Spalte, Absatz 1 *<br>* Seite 11, linke Spalte, letzter Absatz - rechte Spalte, Absatz 1 *<br>* Seite 14, linke Spalte, letzter Absatz - rechte Spalte, Absatz 2 *<br>* Tabelle 5 *<br>* Abbildungen 3,14 *<br>----- | 12<br><br><br><br><br><br><br><br>6 | |
| X | US 2019/254606 A1 (SARHAN SAMEH [US]) 22. August 2019 (2019-08-22)<br>* Absatz [0008] - Absatz [0012] *<br>* Absatz [0027] *<br>* Abbildungen 1,2A,2B *<br>----- | 12 | **RECHERCHIERTE SACHGEBIETE (IPC)**<br><br>A61B |
| Y | US 2011/295127 A1 (SANDLER RICHARD H [US] ET AL) 1. Dezember 2011 (2011-12-01)<br>* Absatz [0025] *<br>----- | 7 | |

-/--

Der vorliegende Recherchenbericht wurde für alle Patentansprüche erstellt

| Recherchenort | Abschlußdatum der Recherche | Prüfer |
|---|---|---|
| Berlin | 9. Juli 2021 | Völlinger, Martin |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
......................................................................
& : Mitglied der gleichen Patentfamilie, übereinstimmendes Dokument

EPO FORM 1503 03.82 (P04C03)

Seite 1 von 2

**Europäisches Patentamt**
**European Patent Office**
**Office européen des brevets**

# EUROPÄISCHER RECHERCHENBERICHT

Nummer der Anmeldung

EP 21 15 6009

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 2016/354063 A1 (WARD III RAYMOND JOSEPH [US] ET AL) 8. Dezember 2016 (2016-12-08) <br> * Absatz [0003] * <br> * Absatz [0020] - Absatz [0021] * <br> * Absatz [0041] * <br> * Abbildung 1 * <br> ----- | 1-12 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Berlin | 9. Juli 2021 | Völlinger, Martin |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

Seite 2 von 2

**ANHANG ZUM EUROPÄISCHEN RECHERCHENBERICHT
ÜBER DIE EUROPÄISCHE PATENTANMELDUNG NR.**

EP 21 15 6009

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-07-2021

| Im Recherchenbericht angeführtes Patentdokument | Datum der Veröffentlichung | Mitglied(er) der Patentfamilie | Datum der Veröffentlichung |
|---|---|---|---|
| DE 102011112226 A1 | 08-05-2013 | KEINE | |
| US 2019254606 A1 | 22-08-2019 | KEINE | |
| US 2011295127 A1 | 01-12-2011 | KEINE | |
| US 2016354063 A1 | 08-12-2016 | US 2016354063 A1<br>US 2019076129 A1 | 08-12-2016<br>14-03-2019 |

Für nähere Einzelheiten zu diesem Anhang : siehe Amtsblatt des Europäischen Patentamts, Nr.12/82

EPO FORM P0461